# EUROPÄISCHE PATENTANMELDUNG

(11) **EP 4 813 304 A1**
(43) Veröffentlichungstag der Anmeldung: **30.09.2026**
(21) Anmeldenummer: 25167064.2
(22) Anmeldetag: 28.03.2025
(51) Int. Cl.: A61B 6/00, A61B 6/03, A61B 6/42

(54) **VERFAHREN ZUR ANSTEUERUNG EINES RÖNTGENBILDGEBUNGSGERÄTS**

(71) Anmelder: Siemens Healthineers AG, 91301 Forchheim (DE)
(72) Erfinder: Faby, Sebastian, 91301 Forchheim (DE); Jürgens, Markus, 91325 Adelsdorf (DE); Lichy, Matthias, 90489 Nürnberg (DE)
(74) Vertreter: Siemens Healthineers Patent Attorneys

(57) **Zusammenfassung**

Die Erfindung betrifft ein Verfahren zur Ansteuerung eines Röntgenbildgebungsgeräts (32) aufweisend zumindest eine Röntgenquelle (37) und in Gegenüberstellung dazu einen photonenzählenden Röntgendetektor (36) zur Akquisition eines dreidimensionalen Objektbilddatensatzes eines Abbildungsbereichs (A) eines Objekts (39) umfassend die Schritte
- Bereitstellen (S1) eines ersten spektralen Bilddatensatzes abbildend zumindest einen Teil des Abbildungsbereichs (A) des Objekts (39),
- Ableiten (S2) einer Einstellung des Röntgenbildgebungsgeräts (32) basierend auf dem ersten spektralen Bilddatensatz für die Akquisition des dreidimensionalen Objektbilddatensatzes,
- Ansteuern (S3) des Röntgenbildgebungsgeräts (32) zur Akquisition des dreidimensionalen Objektbilddatensatzes basierend auf der abgeleiteten Einstellung.

## Beschreibung

Die Erfindung betrifft ein Verfahren zur Ansteuerung eines Röntgenbildgebungsgerät aufweisend zumindest eine Röntgenquelle und in Gegenüberstellung dazu einen photonenzählenden Röntgendetektor zur Akquisition eines dreidimensionalen Objektbilddatensatzes eines Abbildungsbereichs eines Objekts basierend auf einem bereitgestellten ersten spektralen Bilddatensatz. Weiterhin betrifft die Erfindung eine Ansteuerungseinheit zur Ansteuerung eines Röntgenbildgebungsgeräts, ein Röntgenbildgebungsgerät und ein Computerprogrammprodukt.

Unabhängig vom grammatikalischen Geschlecht eines bestimmten Begriffes sind Personen mit männlicher, weiblicher oder anderer Geschlechteridentität mit umfasst.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können. Häufig basieren die bildgebenden Verfahren auf der Erfassung von Röntgenstrahlung, wobei sogenannte Projektionsmessdaten, im Folgenden auch Projektionsdatensätze, erzeugt werden. Beispielsweise können Projektionsmessdaten mit Hilfe eines Computertomographie-Geräts (CT-Gerät) akquiriert werden. Bei CT-Geräten läuft gewöhnlich eine an einer Gantry angeordnete Kombination aus Röntgenquelle und gegenüberliegend angeordnetem Röntgendetektor um einen Messraum um, in dem sich das Untersuchungsobjekt befindet. Das Drehzentrum (auch "Isozentrum" genannt) fällt dabei mit einer sogenannten Systemachse, welche sich in z-Richtung erstreckt, zusammen. Bei einem oder mehreren Umläufen wird das Objekt mit Röntgenstrahlung der Röntgenquelle durchstrahlt, wobei mit Hilfe des gegenüberliegenden Röntgendetektors Bilddatensätze im Form von Projektionsmessdaten erfasst werden. Dies umfassen jeweils eine Mehrzahl an Projektionen für eine Mehrzahl an Winkelrichtungen, welche für die jeweilige Winkelrichtung Informationen über die Schwächung der Strahlung durch das Untersuchungsobjekt enthalten. Basierend auf einem Satz Projektionsmessdaten können dann mittels eines geeigneten Rekonstruktionsalgorithmus dreidimensionale Bilddatensätze für eine räumliche Darstellung des Objekts erzeugt werden.

In der spektralen Computertomographie wie auch in anderen spektralen Bildgebungen werden die unterschiedlichen Absorptionen von Materialen über ein Energiespektrum für weitere Analysen genutzt. Für die spektrale CT-Bildgebung wird in der klinischen Routine dabei beispielsweise die Schwächung bei einer niedrigen und bei einer hohen mittleren Energie der von der Röntgenquelle emittierten Röntgenstrahlung verwendet ("Dual Energy CT"). Unabhängig von der Art der Datenakquisition wird dabei in der Regel im Voraus festgelegt, mit welchen Spannungen die Röntgenröhre bzw. Röntgenröhren emittieren muss bzw. müssen, um eine ausreichende spektrale Separierung zu erreichen. Die transmittierten und geschwächten Röntgenquanten werden somit in zwei Energiebereiche aufgesplittet bzw. wird im Voraus die benötigte Strahlung zur Bildakquisition in einen niedrigen und einen hohen mittleren Energieanteil aufgeteilt und entsprechend Projektionsmessdaten aufgenommen.

Mit der Einführung der photonenzählenden Detektoren in der Computertomographie (photonenzählende CT, engl. photon counting CT - PCCT) kann unter Einsatz eines solchen photonenzählenden Röntgedetektors auch unabhängig von der emittierten Energie der von der Röntgenquelle emittierten Röntgenstrahlung die Akquisition von spektraler Informationen über die erfahrene Schwächung bzw. die Energie der detektierten Röntgenquanten auch bei einer (konstanten) Röhrenspannung erfolgen.

Dabei kann mittels eines geeigneten photonenzählenden Röntgendetektors eine Trennung der detektierten Röntgenquanten in mehrere Energieklassen in Abhängigkeit von im Röntgendetektor eingestellten Energieschwellwerten erfolgen. Allerdings ist auch hier die Energie der dafür nötigen Schwellwerte im Detektor in der Regel im Vorfeld einer Bildakquisition definiert. Daraus kann sich beispielsweise ergeben, dass aufgrund unzureichender Statistik in einzelnen dieser Energieklassen keine ausreichenden Daten für die weitere Analyse für gewisse Operationen, beispielsweise der Jod-Quantifizierung, gewonnen werden oder dass die Schwellwerte nicht optimal über den gesamten Abbildungsbereich des zu akquirierenden Bilddatensatz für eine spätere Analyse gelegt sind. Dies kann zu einer unzureichenden Bildqualität und/oder zu einer vermeidbaren höheren Strahlenbelastungen für einen Patienten führen.

Aufgabe der Erfindung ist es, ein verbessertes Verfahren zur Ansteuerung eines Röntgenbildgebungsgerät, eine entsprechende Ansteuerungseinheit, sowie ein Röntgenbildgebungsgerät und ein Computerprogrammprodukt bereitzustellen, welches zumindest teilweise oben beschriebene Nachteile vermeidet.

Die Aufgabe wird erfindungsgemäß gelöst durch den Gegenstand der unabhängigen Ansprüche. Vorteilhafte Ausführungsformen mit zweckmäßigen Weiterbildungen sind Gegenstand der Unteransprüche.

Die Erfindung betrifft ein Verfahren zur Ansteuerung eines Röntgenbildgebungsgeräts aufweisend zumindest eine Röntgenquelle und in Gegenüberstellung dazu einen photonenzählenden Röntgendetektor zur Akquisition eines dreidimensionalen Objektbilddatensatzes eines Abbildungsbereichs eines Objekts umfassend die Schritte
- Bereitstellen eines ersten spektralen Bilddatensatzes abbildend zumindest einen Teil des Abbildungsbereichs des Objekts,
- Ableiten einer Einstellung zumindest eines Ansteuerungsparameters des Röntgenbildgebungsgeräts basierend auf dem ersten spektralen Bilddatensatz für die Akquisition des dreidimensionalen Objektbilddatensatzes,
- Ansteuern des Röntgenbildgebungsgeräts zur Akquisition des dreidimensionalen Objektbilddatensatzes basierend auf der abgeleiteten Einstellung.

Das Röntgenbildgebungsgerät ist für die Akquisition eines dreidimensionalen Objektbilddatensatzes (3D-Objektbilddatensatz, im Folgenden auch einfach Objektbild-datensatz) ausgebildet. Das Röntgenbildgebungsgerät kann insbesondere als CT-Gerät ausgebildet sein. Es kann beispielsweise auch als C-Bogen-Röntgengerät oder anderweitiges Röntgengerät ausgebildet sein, welches ausgebildet ist einen 3D-Objektbilddatensatz zu erfassen.

Ein 3D-Bilddatensatz erlaubt eine dreidimensionale, insbesondere räumlich dreidimensionale, Darstellung eines Abbildungsbereichs. Der 3D-Bilddatensatz basiert in der Regel auf einer Mehrzahl an erfassten Projektionsdatensätzen, mittels welchen der Abbildungsbereich abgetastet wurde. Basierend auf den erfassten Projektionsdatensätzen und einem geeigneten Rekonstruktionsverfahren, beispielsweise einer gefilterten Rückprojektion, kann dann die räumlich dreidimensionale Darstellung des Abbildungsbereichs erzeugt werden. Ein
3D-Bilddatensatz kann auch als eine Mehrzahl an Schichtbilddatensätzen, sogenannten Querschnittsdarstellungen, dargestellt werden. Ein Schichtbilddatensatz umfasst jeweils eine Schicht des 3D-Bilddatensatz an einer Position entlang einer ausgezeichneten Achse. Ein Schichtbilddatensatz erlaubt jeweils eine zweidimensionale, insbesondere räumlich zweidimensionale, Darstellung der jeweiligen Schicht des 3D-Bilddatensatzes.

Vorteilhafterweise umfasst ein solcher 3D-Bilddatensatz mehrere Voxel, insbesondere Bildpunkte. Dabei kann jedes Voxel vorzugsweise jeweils einen Bildwert, im Folgenden auch Voxelwert, insbesondere einen CT-Bildwert in HU ("Hounsfield Units") im Falle eines CT-Scans oder einen dazu analogen Intensitätswert, aufweisen. Analog dazu kann ein Schichtbilddatensatz mehrere Pixel, insbesondere Bildpunkte, umfassen. Dabei kann jeder Pixel vorzugsweise jeweils einen Bildwert, insbesondere einen CT-Bildwert in HU ("Hounsfield Units") im Falle eines CT-Scans oder eine dazu analogen Intensitätswert, aufweisen.

Ein zweidimensionaler (2D-)Bilddatensatz erlaubt eine zweidimensionale, insbesondere räumlich zweidimensionale, Darstellung eines Abbildungsbereichs. Ein 2D-Bilddatensatz weist insbesondere mehrere Pixel, insbesondere Bildpunkte, auf, wobei jeder Pixel vorzugsweise jeweils einen Bildwert aufweist, welcher die lokal detektierte Intensität der transmittierten Strahlung repräsentiert.

Als photonenzählendender Röntgendetektor kann insbesondere ein photonenzählendender, direkt-konvertierender Röntgendetektoren eingesetzt werden. Eintreffende Röntgenstrahlung bzw. Photonen können in solchen Röntgendetektoren durch ein geeignetes Konvertermaterial in elektrische Pulse umgewandelt werden. Als Konvertermaterial können beispielsweise CdTe, CZT, Hgl2, GaAs oder andere verwendet werden. Die elektrischen Pulse werden von einer Auswerteelektronik, beispielsweise einem integrierten Schaltkreis (Application Specific Integrated Circuit, ASIC), bewertet. In zählenden Röntgendetektoren wird einfallende Röntgenstrahlung dann durch Zählen der elektrischen Pulse, welche durch die Absorption von Röntgenphotonen im Konvertermaterial ausgelöst werden, gemessen. Die Höhe oder auch die Länge eines erzeugten elektrischen Pulses ist in der Regel außerdem proportional zur Energie des absorbierten Röntgenphotons. Dadurch kann eine spektrale Information durch den Vergleich der Höhe bzw. Länge des elektrischen Pulses mit einem Energieschwellwert extrahiert werden. Häufig weisen photonenzählende Röntgendetektor mehrere einstellbare Energieschwellen für einen Vergleich der erzeugten elektrischen Pulse auf, so dass energieaufgelöste Messungen in Abhängigkeit von mehreren durch die Energieschwellwerte definierten Energiebereiche ermöglicht sind.

Insbesondere kann der 3D-Objektbilddatensatz ein spektraler Bilddatensatz sein, insbesondere auf energieaufgelösten Messdaten in Abhängigkeit von mindestens zwei Energieschwellwerten basieren.

Das Objekt kann ein menschlicher und/oder tierischer Patient sein. Es kann jedoch auch ein unbelebtes Objekt, beispielsweise ein Untersuchungsphantom, sein.

Das Bereitstellen eines ersten spektralen Bilddatensatzes, insbesondere spektralen Röntgenbilddatensatzes, kann insbesondere ein Erfassen und/oder Auslesen eines computerlesbaren Datenspeichers und/oder ein Empfangen aus einer Datenspeichereinheit, beispielsweise einer Datenbank, umfassen. Ferner kann der Bilddatensatz von einer Verarbeitungseinheit eines medizinischen Bildgebungsgeräts zur Aufnahme des spektralen Bilddatensatzes bereitgestellt werden. Das Bildgebungsgerät kann beispielsweise eine CT-Gerät und/oder ein medizinisches Röntgengerät, insbesondere ein medizinisches C-Bogen-Röntgengerät umfassen, welches für die Messung von spektral aufgelösten Bilddatensatzen ausgelegt ist, insbesondere einen photonenzählenen Röntgendetektor aufweist. Das Bildgebungsgerät kann vorzugsweise das Röntgenbildgebungsgerät sein, welches mittels des vorgeschlagenen Verfahrens angesteuert werden soll. Es kann jedoch auch ein anderes Bildgebungsgerät sein, insbesondere wenn der erste spektrale Bilddatensatz auf einem zuvor aufgenommenen, spektralen dreidimensionalen Vorbilddatensatz basiert. Das Bereitstellen eines ersten spektralen Bilddatensatzes kann auch ein Erzeugen, insbesondere ein Rekonstruieren, des Bilddatensatzes basierend auf vorerfassten Messdaten umfassen.

Der erste spektrale Bilddatensatz kann ein 2D-Bilddatensatz oder ein 3D-Bilddatensatz umfassen. Er kann auch in Form eines Projektionsdatensatzes vorliegen.

Vorzugsweise liegt das Objekt bei der Erfassung des Daten für den ersten spektralen Bilddatensatz und für die Akquisition des Objektbilddatensatz in unveränderter Lage und Position vor, so dass eine räumliche Übertragung der lokalen Eigenschaften des Objekts besonders vorteilhaft und einfach ermöglicht ist.

Der erste spektrale Bilddatensatz kann spektrale Information in dem Sinne aufweisen, dass der erste spektrale Bilddatensatz auf Messdaten basiert, welche die Eigenschaften des Objekts in Hinblick auf die Absorption von Röntgenstrahlung durch das Objekt spektral aufgelöst, d.h. in Abhängigkeit zumindest zweier mittlerer Energien oder zumindest zweier Energiebereiche der Röntgenstrahlung, repräsentieren. Bevorzugt ist der erste spektrale Bilddatensatz mittels eines photonenzählenden Röntgendetektors und in Abhängigkeit zumindest zweier Energieschwellwerte erfasst worden. Basierend darauf kann auf eine Materialeigenschaft des Untersuchungsobjekts, beispielsweise eher "wasserartig" oder eher "jodartig", geschlossen werden. Beispielsweise kann ein Verfahren der Materialzerlegung auf die dem ersten spektralen Bilddatensatz zugrunde liegenden Daten angewandt worden sein. Basierend auf dem spektral aufgelösten ersten Bilddatensatz kann verbessert auf eine erwartete spektrale Absorption von Röntgenstrahlung, inbesondere eine Abschwächung und/oder Strahlaufhärtung durch das Objekt, und/oder zu erwartenden mittels des Röntgendetektors gemessenen Statistik bei der Durchleuchtung des Objekts mit Röntgenstrahlung bei der Akquisition des Objektbilddatensatzes mittels des photonenzählenden Röntgendetektors, insbesondere auch ortsaufgelöst, geschlossen werden. Der erste spektrale Bilddatensatz ermöglich damit Zugang zu einer vorteilhaft detaillierten, spektralen Objektinformation, welche im Schritt des Ableitens berücksichtigt werden kann.

Der erste spektrale Bilddatensatz stellt spektral aufgelöste Informationen zumindest für einen Teil des Abbildungsbereichs zur Verfügung. Der erste spektrale Bilddatensatz kann spektral aufgelöste Informationen für den gesamten Abbildungsbereich oder auch für das gesamte Untersuchungsobjekt bereitstellen.

Basierend auf dem ersten spektralen Bilddatensatz kann eine vorteilhafte Einstellung des zumindest einen Ansteuerungsparameter abgeleitet werden, welcher für die Ansteuerung des Röntgenbildgebungsgeräts zur Akquisition des 3D-Objektbilddatensatzes eingesetzt wird. Die Einstellung kann für den gesamten Abbildungsbereich, lediglich für den Teil des Abbildungs-bereichs, welcher vom ersten spektralen Bilddatensatz umfasst ist oder für einen zweiten Teil des Abbildungsbereichs abgleitet werden, welcher ortsnah zum vom ersten spektralen Bilddatensatz umfassten ersten Teil liegt. Derart kann erwartet werden, dass sich die Eigenschaften des Objekts zwischen diesem ersten Teil und diesem zweiten Teil nur unwesentlich unterscheiden oder diese zumindest abgeleitet werden können. Beispielsweise kann der zweite Teil örtlich direkt anschließend an den ersten Teil liegen. Beispielsweise kann der erste spektrale Bilddatensatz einen während der Akquisition des dreidimensionalen Objektbilddatensatzes erfassten spektralen Projektionsdatensatz umfassen. Beispielsweise kann eine Einstellung abgeleitet werden für die Akquisition eines zeitlich nachfolgenden, beispielsweise des direkt danach folgenden oder des wiederum darauf folgenden Projektionsdatensatzes, welcher einen örtlich nachfolgenden Teilbereich des Abbildungsbereichs abbildet. Erfolgt das Ableiten lediglich für einen Teil des Abbildungsbereichs, so kann bevorzugt ein wiederholtes Ableiten für einen weiteren Teil im Rahmen des Verfahrens stattfinden, so dass insgesamt für den gesamten Abbildungsbereich die Akquisistion des Objektbilddatensatzes eine abgeleitete Einstellung für den zumindest einen Ansteuerungsparameter vorliegt. Beispielsweise kann wiederholt ein Ableiten basierend auf während der Akquisition des dreidimensionalen Objektbilddatensatzes erfassten spektralen Projektionsdatensätzen für jeweils nachfolgende Projektionsdatensätzen erfolgen.

Die abgeleitete Einstellung für den zumindest einen Ansteuerungsparameter kann einen festen Wert oder auch einen Werteverlauf für den Ansteuerungsparameter umfassen, welcher bei der Akquisition des Objektbilddatensatzes angewandt werden soll und entsprechend bei der Ansteuerung des Röntgenbildgebungsgeräts berücksichtigt wird.

Der Ansteuerungsparameter kann insbesondere ein Ansteuerungsparameter der Röntgenröhre oder des Röntgendetektors umfassen. Er kann auch einen anderweitigen Ansteuerungsparameter umfassen. Insbesondere kann der zumindest eine Ansteuerungsparameter einen solchen Parameter umfassen, welcher direkten Einfluss auf eine im Röntgendetektor gemessene Statistik und/oder die spektrale Separierung und/oder die gemessene spektrale Information hat. Insbesondere können auch Einstellungen mehrere Ansteuerungsparameter abgeleitet werden. Gemäß einem Aspekt des vorgeschlagenen Verfahrens umfasst der zumindest eine Ansteuerungsparameter zumindest einen Energieschwellwert des photonenzählenden Röntgendetektors für die Akquisition des Objektbilddatensatzes. Vorteilhaft können die Energieschwellwerte des Röntgendetektors basierend auf der spektralen Information des ersten spektralen Bilddatensatzes für die Akquisition des Objektbilddatensatzes optimiert eingestellt werden und, beispielsweise, in Abhängigkeit der Energieschwellwerte jeweils eine ausreichende detektierte Statistik gewährleistet werden. Es können auch andere Parameter des Röntgendetektors umfasst sein, beispielsweise eine Signalverstärkung oder eine Pulsformungszeit für das im Detektor als Reaktion auf ein detektiertes Röntgenphoton erzeugte elektrische Signal.

Der zumindest eine Ansteuerungsparameter kann auch eine Röhrenspannung, einen Röhrenstrom oder eine Vorfilterung der Röntgenquelle und/oder eine Rotationszeit oder einen relativen Vorschub ("pitch") zwischen Objekt und Röntgendetektor während der Akquisition des Objektbilddatensatzes umfassen. Darüber hinaus kann es auch weitere Parameter des Röntgenbildgebungsgeräts geben, welche als im Rahmen des vorgeschlagenen Verfahrens abgeleitet werden können. Beispielsweise kann auch die Verwendung eines zusätzlichen Spektrums als Ansteuerungsparameter miteingehen.

Das Ableiten der Einstellung des zumindest einen Ansteuerungsparameters basierend auf dem ersten spektralen Bilddatensatz ermöglicht das Bestimmen, insbesondere Abschätzen, einer erwarteten spektralen Absorption bei der Akquisition des Objektbilddatensatzes, insbesondere eine spektral aufgelöste Schwächung und/oder Strahlaufhärtung durch das aufzunehmende Objekt. Insbesondere kann basierend darauf eine optimierte Einstellung des zumindest einen Ansteuerungsparameters für die Akquisition des Objektbilddatensatzes, beispielsweise vorteilhaft auch ortsaufgelöst über den Abbildungsbereich, abgeleitet werden. Insbesondere kann dabei eine erwartete gemessene Statistik im Röntgendetektor für die Akquisition des Objektbilddatensatz, insbesondere auch ortsaufgelöst über den Abbildungsbereich, verbessert abgeschätzt werden und darauf basierend die Einstellung des zumindest einen Ansteuerungsparameters optimiert werden. Eine optimierte Einstellung kann insbesondere darauf abzielen, dass für eine spätere Analyse und/oder Weiterverarbeitung des Objektbilddatensatzes eine geeignete gemessene Statistik im Röntgendetektor und/oder Bildqualität, beispielsweise quantifiziert durch einen Signal-zu-Rausch-Verhältnis, im Objektbilddatensatz vorliegt. Weiterhin kann eine erwartete Strahlenbelastung durch die Akquisition des Objektbilddatensatzes einbezogen werden. Beim Ableiten kann vorzugsweise neben der durch den ersten spektralen Bilddatensatz zur Verfügung gestellten Objektinformation außerdem die dem Objektbilddatensatz zugrunde liegende klinische Bildgebungsanwendung bzw. der zugrunde liegende Bildgebungsgrund miteinbezogen werden. Für das Ableiten können simulierte oder gemessene Modelle und/oder historische Bildakquisitionen an einer Patientenpopulation für die Akquisition des Objektbilddatensatzes in Bezug auf den zumindest einen Ansteuerungsparameter einbezogen werden. Dabei ist auch denkbar, dass beim Ableiten eine Methode der künstlichen Intelligenz, beispielsweise ein neuronales Netz, insbesondere ein tiefes neuronales Netz, eingesetzt wird.

Basierend auf der abgeleiteten Einstellung wird das Röntgenbildgebungsgerät anschließend für die Akquisition des Objektbilddatensatzes angesteuert. Beispielsweise kann ein Steuersignal für das Röntgenbildgebungsgerät und/oder einzelne Komponenten davon bereitgestellt werden, welches das Röntgenbildgebungsgerät und/oder seine Komponenten in Bezug auf den Ansteuerungsparameter gemäß der abgeleiteten Einstellung anpasst. Vorteilhaft kann ein Objektbild-datensatz akquiriert und anschließend bereitgestellt werden, welcher günstige Bildeigenschaften, insbesondere eine günstige Bildqualität, für eine darauf basierende Analyse und/oder Weiterverarbeitung aufweist. Dies kann insbesondere auch die Vermeidung einer unnötigen Strahlenbelastung ermöglichen.

Im Rahmen des Verfahrens kann auch ein zweiter oder dritter spektraler Bilddatensatz bereitgestellt werden, basierend auf welchem erneut ein Ableiten und damit eine Anpassung der Einstellung des zumindest einen Ansteuerungsparameters durchgeführt wird, wobei das Röntgenbildgebungsgerät dann basierend auf der angepassten Einstellung angesteuert wird. Dies kann beispielsweise im Rahmen einer Perfusionsbildgebung zum Tragen kommen, wobei vor der Akquisition des eigentlichen Objektbilddatensatzes wiederholt ein Topogram aufgenommen werden kann, um den optimalen Zeitpunkt der Messdatenaufnahme zu bestimmen. Dies kann beispielsweise umfassen, wiederholt basierend auf während der Akquisition des dreidimensionalen Objektbilddatensatzes erfassten spektralen Projektionsdatensätzen ein Ableiten der Einstellung für zeitlich darauf folgende erfasste Projektionsdatensätze vorzunehmen.

Gemäß einem Aspekt der Erfindung kann der erste spektrale Bilddatensatz ein spektrales Topogramm, einen zuvor aufgenommenen, spektralen, dreidimensionalen Vorbilddatensatz und/oder einen während der Akquisition des dreidimensionalen Objektbilddatensatzes erfassten spektralen Projektionsdatensatz umfassen.

Vor dem Aufnehmen von Projektionsdaten für einen dreidimensionalen Bilddatensatz, insbesondere CT-Bilddatensatz, wird häufig ein, in den meisten Fällen zweidimensionaler, Übersichtsbilddatensatz des Untersuchungsobjekts erzeugt, ein sogenanntes Topogramm. In der klinischen Routine liegt das Topogramm ohne eine spektrale Information vor. Dieses kann beispielsweise dazu dienen den Aufnahmebereich und damit den Darstellungsbereich für den nachfolgenden dreidimensionalen Bilddatensatz festzulegen. Ein solcher zweidimensionaler Übersichtsbilddatensatz kann beispielsweise in einem CT-Gerät erzeugt werden, indem das Untersuchungsobjekt entlang der Rotationsachse der Röntgenquelle lateral kontinuierlich verschoben wird, während unter festen Projektionswinkel Messdaten aufgenommen werden, ohne dass die Röntgenquelle und der Röntgendetektor während der Aufnahme um das Untersuchungsobjekt rotiert wird. Auf Basis dieser Messdaten kann dann ein Übersichtsbilddatensatz des Untersuchungsobjekts zusammengefügt und dargestellt werden. Besonders vorteilhaft kann mittels eines photonenzählenden Röntgendetektors nun ein spektrales Topogramm aufweisend spektrale Informationen für das vorgeschlagene Verfahren bereitgestellt werden. Dieses kann weiterhin vorteilhaft spektrale Information über den gesamten Abbildungsbereich zur Verfügung stellen, so dass das Ableiten der Einstellung des zumindest einen Ansteuerungsparameters für den gesamten Abbildungsbereich ermöglicht werden kann. Weiterhin wird ein Topogramm in der Regel vor einer jeweiligen Akquisition des Objektbilddatensatzes durchgeführt, so dass keine zusätzliche Strahlenbelastung notwendig ist, wobei insbesondere ein photonenzählender Röntgendetektor vorteilhaft die direkte Gewinnung der spektralen Information basierend auf mehreren Energieschwellwerten ohne eine zusätzliche Strahlenbelastung ermöglichen kann. So kann beispielsweise auf die Anwendung zweier verschiedener Röhrenspektren verzichtet werden.

Auch ein zuvor aufgenommener, spektraler dreidimensionaler Vorbilddatensatz des Objekts kann für das Ableiten herangezogen werden. Ein Vorbilddatensatz kann ein historische Bilddatensatz des Objekt umfassen. Vorzugweise ist die Akquisition des Vorbilddatensatzes jedoch in direkter zeitlicher Nähe zum Objektbilddatensatz und unter Beibehaltung der Objektlage und Position erfolgt, so dass eine hinreichende Übereinstimmung der Objekteigenschaften gewährleistet ist. Eine dreidimensionale Information kann eine noch detaillierte Ableitung der Einstellung des zumindest einen Ansteuerungsparameters erlauben.

Auch ein während der während der Akquisition des dreidimensionalen Objektbilddatensatzes erfasster spektraler Projektionsdatensatz kann für ein Ableiten herangezogen werden. Dies kann eine vorteilhafte stets den aktuellen Gegebenheiten und Messwerten angepasste Ableitung für folgende Projektionsdatensätze und damit eine hoch dynamische Modulation während der Akquisition des Objektbilddatensatz ermöglichen.

Weiterhin kann der erste spektrale Bilddatensatz auf energieaufgelösten Messdaten basierend auf mindestens drei Energieschwellwerten basieren. Vorteilhaft kann eine hohe spektrale Auflösung und damit detaillierte Objektinformation bereitgestellt werden, welche das Ableiten einer vorteilhaften Einstellung verbessert ermöglicht.

Beispielsweise können mehr Materialien unterschieden werden oder die Energieschwellwerte vorteilhaft gezielt eingesetzt werden, dass diese hilfreich für die Lokalisiation spezieller Materialien sind. Beispielsweise könnten Implantate im Objekt, beispielsweise Goldfüllungen in Zähnen, erleichtert lokalisiert werden. Auch wäre beispielsweise eine Lokalisierung von Materialien mit einer k-Kante in einem bestimmten Energiebereich denkbar, z.B. Tracer-Materialien oder speziell markierte Medikamente mit k-Kanten in einem Bereich von 70-100keV. In einer vorteilhaften Variante basiert der erste spektrale Bilddatensatz außerdem auf der gleichen Anzahl Energieschwellwerten wie der zu akquirierende Objektbilddatensatz, so dass eine Ableitung einer vorteilhaften Einstellung der Energieschwellwerte besonders einfach aus dem ersten spektralen Bilddatensatz übertragbar ist. Derart müssen weniger Annahmen in das Ableiten eingehen.

Gemäß einem Aspekt des vorgeschlagenen Verfahrens bezieht das Ableiten die dem Objektbilddatensatz zugrunde liegende klinische Bildgebungsanwendung und/oder den Bildgebungsgrund ein.

Vorteilhafterweise kann die erwartete Zielsetzung und ggf. mit der Bildgebungsanwendung einhergehende Randbedingungen der durchgeführten Bildgebung in das Ableiten der Einstellung des zumindest einen Ansteuerungsparameters eingehen und daraufhin optimiert werden. Beispielsweise soll ein Thorax/Oberbauch-Scan, ein Lungenscan oder eine Hirnperfusionsbildgebung durchgeführt werden, welche jeweils mit unterschiedlichen Zielsetzungen und Bedingungen, beispielsweise den Einsatz von Kontrastmittel, die Ansteuerung der Röntgenröhre hinsichtlich des emittierten Energiespektrums oder die geplante Weiterverarbeitung der Bilddaten hinsichtlich einer auf den Bilddaten basierenden Diagnose, einhergehen. Beispielsweise kann für die, insbesondere auch spektrale, Bildgebung eines bestimmten Zielorgans die Einstellung einen anderen Wert oder Werteverlauf umfassen als in Bereichen abseits des Zielorgans, so dass eine gezielte Analyse des Zielorgans vorteilhaft ermöglicht ist, wobei abseits des Zielorgans andere Werte oder ein anderer Werteverlauf des Ansteuerungsparameters zu vorteilhaften Bildergebnissen führt, eine anderweitige Analyse ermöglicht oder zu einer geringeren, notwendigen Strahlenbelastung führt. Vorteilhafterweise wird nicht nur eine Objektinformation, sondern auch die Anwendungsinformation beim Ableiten miteinbezogen. Vorteilhaft kann eine auf das Objekt und die Anwendung hin ausgerichtete Statistik erreicht werden. Dadurch kann eine unnötige Strahlenbelastung und/oder unzureichende Bildqualität verbessert vermieden werden.

Wie bereits zuvor beschrieben, kann die abgeleitete Einstellung einen festen Wert für den zumindest einen Ansteuerungsparameter umfassen. Der eine Wert kann für den gesamten Abbildungsbereich festgelegt sein. Beispielsweise kann basierend auf den Informationen des ersten spektralen Bilddatensatzes ein fester Satz von Energieschwellwerten für den Röntgendetektor für den gesamten Abbildungsbereich festgelegt werden, welcher berücksichtigt, dass im gesamten Abbildungsbereich eine geeignete Statistik in Abhängigkeit der Energieschwellwerte am Röntgendetektor gewährleistet ist. Dies kann genauso auch auf einen anderen Ansteuerungsparameter übertragen werden.

Gemäß einem weiteren Aspekt der Erfindung kann der zumindest eine Ansteuerungsparameter jedoch während der Akquisition des dreidimensionalen Objektbilddatensatzes unterschiedliche Werte aufweisen.

Dies kann insbesondere umfassen, dass der zumindest eine Ansteuerungsparameter zumindest basierend auf dem durch den ersten spektralen Bilddatensatz bereitgestellte Information während der Akquisition des Objektbilddatensatzes moduliert wird. Die Einstellung des zumindest einen Ansteuerungsparameters kann dadurch vorteilhaft auf die örtlichen Begebenheiten im Abbildungsbereich abgestimmt werden. Vorteilhafterweise wird auf die Eigenschaften des Objekts und ggf. auch die Randbedingungen der Bildgebungsanwendung ortsaufgelöst berücksichtigt, so dass ein vorteilhafter Objektbilddatensatz gewährleistet werden kann. Beispielsweise kann basierend auf dem ersten spektralen Bilddatensatz ein Werteverlauf für den zumindest einen Ansteuerungsparameter über den Abbildungsbereich abgeleitet werden, basierend auf welchem das Röntgengerät bei der Akquisistion des Objektbilddatensatzes angesteuert wird. Derart kann ortsaufgelöst eine verbesserte Datenakquise und damit eine verbesserte Bildqualität des Objektbilddatensatz gewährleistet werden.

Gemäß einer Ausführungsvariante davon kann der Abbildungsbereich in zumindest zwei Regionen aufgeteilt sein und der zumindest eine Ansteuerungsparameter für die zumindest zwei Regionen unterschiedliche Werte annehmen. Insbesondere können auch mehr als zwei Regionen unterschieden und der zumindest eine Ansteuerungsparameter auf mehr als zwei Werte eingestellt werden.

Die Regionen können in besonders einfacher Weise basierend auf einem spektralen Topogramm oder einem spektralen Vorbilddatensatz vorab bestimmt werden. Insbesondere können Regionen entlang einer Längsachse des Röntgenbildgebungsgeräts unterschieden werden. Die Längsachse kann insbesondere dadurch ausgezeichnet sein, dass eine Systemachse des Röntgenbildgebungsgeräts, insbesondere die Rotationsachse eines CT-Geräts oder eines C-Bogen-Röntgengeräts für die Akquisition des Objektbilddatensatzes parallel dazu verläuft. Insbesondere können solche Regionen unterschieden werden, in welchen sich die spektrale Absorption voneinander unterscheidet, so dass ein angepasster Ansteuerungsparameter zu vorteilhaften Ergebnissen führen kann. Weiterhin kann für unterschiedliche Regionen eine unterschiedliche Zielsetzung der Anwendung und damit ein anderer optimierter Wert für den Ansteuerungsparameter einhergehen. Beispielsweise unterscheidet sich der Wert für den zumindest einen Ansteuerungsparameter in einem Bereich mit einem hohen Kochenanteil von einer Region abseits davon. Beispielsweise kann für die, insbesondere auch spektrale, Bildgebung eines bestimmten Organs ein anderer Wert für den Ansteuerungsparameter, beispielsweise andere Energieschellwerte, vorteilhaft sein als für eine Region abseits davon. Beispielsweise ist für eine Zielregion für eine geplante Weiterverarbeitung der Bilddaten hinsichtlich der auf den Bilddaten basierenden Diagnose ein anderer Wert für den zumindest einen Ansteuerungsparameter vorteilhaft, wohingegen abseits davon ein anderer Ansteuerungsparameter gewählt wird.

Vorteilhaft kann eine ortsaufgelöste Anpassung des zumindest einen Ansteuerungsparameters gewährleistet werden, wobei gleichzeitig eine einfache Ausführbarkeit erreicht werden kann. Die Ortsauflösung der Modulation des Ansteuerungsparameters ist dabei abhängig von der Art und Anzal der gewählten Regionen.

Daneben ist auch denkbart, dass ein kontinuierlicher Werteverlauf in Abhängigkeit der mittels des ersten spektralen Bilddatensatzes bereitgestellten Information für den zumindest einen Ansteuerungsparameter abgeleitet wird und derart eine kontinuierliche Anpassung des Ansteuerungsparameters während der Akquisition des Objektbilddatensatzes erreicht wird. Beispielsweise können Energieschwellwerte kontinuierlich während der Akquisition des Objektbilddatensatzes angepasst werden.

Gemäß einem Aspekt der Erfindung kann für die Akquisition des dreidimensionalen Objektbilddatensatzes eine Mehrzahl an Projektionsbilddatensätzen erfasst werden, wobei der Wert des zumindest einen Ansteuerungsparameter von Projektionsdatensatz zu Projektionsdatensatz angepasst wird.

Das Anpassen kann dann umfassen, dass für jeden Projektionsdatensatz ein vorteilhafter Wert für den zumindest einen Ansteuerungsparameter abgeleitet wird. Ein Anpassen kann dabei aber umfassen, dass der angepasste Wert des Ansteuerungsparameter gleich dem Wert des Projektiondatensatzes davor ist insofern ein gleicher Wert abgeleitet wurde. Dies kann insbesondere umfassen, dass das Ableiten jeweils auf einem während der Akquisition des Objektbilddatensatzes erfassten Projektionsdatensatz basiert. Vorteilhaft wird eine starke ortsabhängige und/oder zeitliche Auflösung der Einstellung des zumindest einen Ansteuerungsparameters in Abhängigkeit der spektralen Information des ersten spektralen Bilddatensatzes erreicht und eine hochdynamische Modulation ermöglicht.

Gemäß einem weiteren Aspekt der Erfindung kann der zumindest eine Ansteuerungsparameter auch während einer Erfassung eines vom Objektbilddatensatz umfassten Projektionsdatensatzes unterschiedliche Werte aufweisen.

Beispielsweise kann der zumindest eine Ansteuerungsparameter auch während der Erfassung eines Projektionsdatensatzes variieren. Beispielsweise kann je nach relativer Position der Strahlungsquelle bzw. des zugehörigen Röntgendetektors und des Objekts in radialer Richtung eine optimierte Einstellung des zumindest einen Ansteuerungsparameters erreicht werden. Beispielsweise kann eine seitliche Durchleuchtung von einer fronalen Durchleuchtung des Objekts unterschieden werden und eine geeignete Anpassung des zumindest einen Ansteuerungsparameters erreicht werden. Vorteilhaft kann eine hohe Bildqualität bei ggf. geringere Stahlenbelastung erreicht werden.

Gemäß einem weiteren Aspekt der Erfindung umfasst der zumindest eine Ansteuerungsparameter einen Parameter des Röntgendetektors und der photonenzählende Röntgendetektor eine matrixartige Anordnung einer Mehrzahl an Pixelelementen, wobei die Einstellung des zumindest einen Ansteuerungsparameters für Pixelelemente der Mehrzahl an Pixelelementen zumindest teilweise unterschiedlich ist.

Inbesondere kann dies umfassen, dass der Ansteuerungparameter für die Pixelelemente der Mehrzahl an Pixelelementen des photonenzählenden Röntgendetektors räumlich getrennt voneinander eingestellbar und ggf. entsprechend räumlich getrennt auch während der Akquisition des Objektbilddatensatzes anpassbar ist. Beispielsweise umfasst der Ansteuerungsparameter, dass für jedes Pixelelement der Mehrzahl an Pixelelementen eine Anzahl an einstellbaren Energieschwellwerten vorliegt, wobei im Schritt des Ableitens für verschiedene Pixelelemente der Mehrzahl verschiedene Einstellungen für die Anzahl an Energieschwellwerten für die Akquisition des Objektbilddatensatzes abgeleitet werden. Im Schritt des Ansteuerns können die Pixelelemente der Mehrzahl dann basierend darauf entsprechend der abgeleiteten Einstellung unterschiedlich angesteuert werden.

Dies kann umfassen, dass eine Ableitung jeweils pixelindividuell, d.h. für jedes Pixelelement separat, vorliegt und der Ansteuerungsparameter ggf. auch im Rahmen des vorgeschlagenen Verfahrens während der Akquisition des Objektbilddatensatzes pixelindividuell unterschiedliche Werte aufweist und/oder pixelindviduell angepasst wird. Dies kann auch umfassen, dass Pixelelemente in Gruppen, beispielsweise zeilenweise, zusammengefasst werden, wobei eine Ableitung jeweils für die Gruppe an Pixelelementen zusammen erfolgt und der Ansteuerungsparameter ggf. auch im Rahmen des vorgeschlagenen Verfahrens während der Akquisition des Objektbilddatensatzes gruppenweise unterschiedliche Werte aufweist und/oder entsprechend gruppenweise angepasst wird. Die Gruppen können auch anderweitig zusammengefasst sein, beispielweise mittig im Röntgendetektor liegende Pixelelemente und randseitig liegende Pixelelemente oder auch anderweitig.

Dies kann insbesondere umfassen, dass für den zumindest einen Ansteuerungsparameter innerhalb der Pixelmatrix variierende Einstellungen und darüberhinaus auch eine jeweilige zeitliche Modulation der Werte während der Akquisition des Objektbilddatensatzes erfolgt. Beispielsweise kann dies umfassen, dass eine Einstellung der Anzahl an Energieschwellwerte während der Akquisition des Objektbilddatensatzes innerhalb der Matrix an Pixelelemente kontinuierlich und insbesondere auch in Abhängigkeit der jeweils relativen örtlichen Position des Pixelelements zum Objekt verschoben werden. Vorteilhaft erfolgt eine höchstindividuelle Einstellung des Ansteuerungsparameters.

Gemäß einer vorteilhaften Ausbildung davon kann die Einstellung des zumindest einen Ansteuerungsparameters entlang einer Längsachse des Röntgenbildgebungsgeräts, insbesondere zeilenweise oder zeilengruppenweise, innerhalb der matrixartigen Anordnung der Mehrzahl an Pixelelementen variieren. Dies umfasst insbesondere zumindest eine zeilenweise oder zeilengruppenweise (d.h. jeweils eine Mehrzahl an Zeilen) Zusammenfassung der Pixelelemente der Pixelmatrix hinsichltich der Einstellung des zumindest einen Ansteuerungsparameters. Innerhalb der Zeile oder Zeilengruppe kann dann jeweils die gleiche Einstellung für die Pixelelemente abgeleitet sein, wobei sie sich zeilenweise oder zeilengruppenweise entsprechend unterscheiden kann. Dies kann eine vorteilhafte Reduzierung der Komplexität im Gegensatz zu einer pixelindividuellen Ableitung darstellen.

Gemäß einem weiteren Aspekt der Erfindung weist der photonenzählende Röntgendetektor entlang einer Längsachse des Röntgenbildgebungsgeräts eine Ausdehnung von mindestens 6 cm auf.

Durch die großflächige Ausdehnung kann eine schnelle Datenakquise über den Abbildungs-bereich ermöglicht werden. Inbesondere bei eine Anwendung in Zusammenhang mit bewegten Strukturen kann dies besonders vorteilhaft, auch beispielsweise zur Erfassung zeitaufgelöster Bilddatensätze, eingesetzt werden. Diese Umsetzung ist im Rahmen des vorgeschlagenen Verfahrens besonders vorteilhaft in Verbindung mit dem zuvor beschriebenen Aspekt einer variierenden Einstellung innerhalb der Pixelmatrix. Insbesondere beim Einsatz großflächiger Röntgendetektoren, insbesondere einer größeren Ausdehnung entlang der Längsachse des Röntgenbildgebungsgeräts, ist eine räumlich getrennte Einstellung innerhalb der Pixelmatrix besonders vorteilhaft für die Akquisition des Objektbilddatensatzes einsetzbar. Solche Röntgendetektoren überstrecken bei der Akquisition jeweils einen Bereich des Objekts, in welchem in unterschiedlichen Teilbereichen bereits deutlich unterschiedliche Objekteigenschaften vorliegen können. Eine räumlich getrennte Ableitung und Einstellbarkeit ermöglicht vorteilhaft dem Rechnung zu tragen und eine gezielte und optimierte Einstellung des zumindest einen Ansteuerungsparameter über den Röntgendetektor zu gewährleisten.

Gemäß einem weiteren Aspekt der Erfindung kann das Röntgenbildgebungsgerät für die Akquisition des Objektbilddatensatzes in einem sequentiellen Scanmodus betrieben werden, wobei der zumindest eine Ansteuerungsparameter an einer ersten relativen Position zwischen Objekt und Röntgendetektor entlang einer Längsachse des Röntgenbildgebungsgeräts und an einer zweiten relativen Position zwischen Objekt und Röntgendetektor entlang der Längsachse unterschiedliche Werte aufweist.

Ein sequentieller Scanmodus unterscheidet sich in der Regel von einer Spiralabtastung insbesonders dahingehend, dass während der Erfassung eines Projektionsdatensatzes, d.h. beispielsweise während eines Umlaufs der Röngenquelle/des Röntgendetektor um das Objekt, keine gleichzeitige, kontinuierliche relative Bewegung zwischen Objekt und Röntgenquelle/Röntgendetektor stattfindet. Stattdessen erfolgt eine relative Bewegung zur Erfassung eines größeren Abbildungsbereichs zwischen den Akquisitionen der Projektionsdatensätze.

Dieser Aspekt ist insbesondere vorteilhaft in Verbindung mit dem zuvor beschriebenen Aspekt eine größeren Ausdehnung des Röntgendetektors entlang der Längsachse des Röntgenbildgebungsgeräts, da durch die größere Abdeckung durch den Röntgendektor dennoch eine schnelle Datenakquise ermöglicht ist. Bei einer Kombination mit einer räumlich aufgelösten Einstellbarkeit des zumindest einen Ansteuerungsparameters innerhalb der Pixelmatrix des Röntgendetektors lässt sich durch einen sequentiellen Scanmodus außerdem beispielsweise eine vereinfachte Zuordnung und Bildrekonstruktion trotz der erhöhten Komplexität der Messdaten im Gegensatz, beispielsweise, zu einer Kombination mit einer Spiralabtastung erreichen, da hier nicht zusätzlich außerdem ein kontinuierlicher Verschub des Objekts berücksichtigt werden muss.

Desweiteren ist im Rahmen des vorgeschlagenen Verfahrens auch denkbar, dass die Informationen des spektralen Bilddatensatzes auch in einen der Akquise der Messdaten nachgelagerten Verabreitungsschritte eingeht. Beispielsweise kann auch eine Gewichtung von Messdaten, welche in Abhängigkeit mehrere Energieschwellwerte erfasst wurden, basierend auf dem ersten spekralen Bilddatensatz abgeleitet werden.

Die Erfindung betrifft außerdem eine Ansteuerungseinheit zur Ansteuerung eines Röntgenbildgebungsgeräts aufweisend zumindest eine Röntgenquelle und in Gegenüber-stellung dazu einen photonenzählenden Röntgendetektor zur Akquisition eines dreidimensionalen Objektbilddatensatzes eines Abbildungsbereichs eines Objekts, welche zur Ausführung eines zuvor beschriebenen Verfahrens nach einem der vorangehenden Ansprüche ausgebildet ist.

Die Vorteile der vorgeschlagenen Ansteuerungseinheit entsprechen im Wesentlichen den Vorteilen des vorgeschlagenen Verfahrens zur Ansteuerung eines Röntgenbildgebungsgeräts. Hierbei erwähnte Merkmale, Vorteile oder alternative Ausführungsformen können ebenso auch auf die anderen beanspruchten Gegenstände übertragen werden und umgekehrt.

Die Ansteuerungseinheit kann eine Recheneinheit, eine Speichereinheit und/oder eine Schnittstelle umfassen. Sie kann außerdem eine Steuereinheit umfassen. Die Ansteuerungseinheit, insbesondere die Komponenten der Ansteuerungseinheit, kann bzw. können zum Ausführen der einzelnen Schritte des vorgeschlagenen Verfahrens und der zuvor beschriebenen Varianten ausgebildet sein. Die Schnittstelle kann zum Bereitstellen des ersten spektralen Bilddatensatzes ausgebildet sein. Die Recheneinheit und/oder die Speichereinheit können ausgebildet sein zum Ableiten einer Einstellung zumindest eines Ansteuerungsparameters des Röntgendetektors und/oder der Röntgenquelle basierend auf dem ersten spektralen Bilddatensatz für die Akquisition des dreidimensionalen Objektbilddatensatzes. Die Steuereinheit kann ausgebildet sein zum Ansteuern des Röntgenbildgebungsgeräts. Die Steuereinheit kann zum Bereitstellen eines Steuersignals an das Röntgenbildgebungsgeräts ausgebildet sein, basierend auf der abgeleiteten Einstellung, so dass das Röntgenbildgebungsgerät während der Akquisistion des Objektbilddatensatzes entsprechend der abgeleiteten Einstellung betrieben wird. Die Steuereinheit kann beispielsweise auch separat von den anderen beschriebenen Komponenten ausgebildet sein, wobei mittels der Schnittstelle ein Ergebnis des Schritts des Ableitens an die Steuereinheit ausgegeben werden kann, worauf basierend mittels der Steuereinheit eine Steuerung des Röntgenbildgebungsgeräts umgesetzt wird.

Die Ansteuerungseinheit kann außerdem eine Benutzerschnittstelle in Form einer Darstellungseinheit umfassen, welche ausgebildet ist, den ersten spektralen Bilddatensatz und/oder den Objektbilddatensatz oder Schnittbilddarstellungen davon darzustellen. Dies kann in Form eines Monitors, einen Touchscreens oder eines anderweitigen, geeigneten Anzeigegeräts umgesetzt sein. Die Ansteuerungseinheit kann außerdem eine Benutzerschnittstelle in Form einer Eingabeeinheit umfassen, welche ausgebildet ist, eine manuelle Benutzereingabe zu ermöglichen. Dies kann beispielsweise mittels einer Tastatur und Maus mit entsprechender Eingabemöglichkeit oder mittels einer anderweitigen, geeigneten Eingabeeinheit ermöglicht sein. Ebenso kann die Eingabeeinheit in die Darstellungseinheit integriert sein, beispielsweise in Form eines kapazitiven und/oder resistiven Eingabedisplays. Dabei kann die Eingabeeinheit außerdem durch Eingabe eines Nutzers eine Steuerung des vorgeschlagenen Verfahrens zur Ansteuerung eines Röntgenbildgebungsgeräts ermöglicht werden.

Die Erfindung betrifft außerdem ein Röntgenbildgebungsgerät umfassend eine zuvor beschriebene Ansteuerungseinheit.

Die Vorteile des vorgeschlagenen Röntgenbildgebungsgerät entsprechen im Wesentlichen den Vorteilen des vorgeschlagenen Verfahrens und/oder der vorgeschlagenen Ansteuerungseinheit. Hierbei erwähnte Merkmale, Vorteile oder alternative Ausführungsformen können ebenso auch auf die anderen beanspruchten Gegenstände übertragen werden und umgekehrt.

Das Röntgenbildgebungsgerät kann vorzugsweise als CT-Gerät ausgebildet sein. Es kann aber auch ein anderweitiges medizinisches Röntgengerät, welches zur Akquisition eines dreidimensionalen Objektbilddatensatzes ausgebildet ist, insbesondere beispielsweise ein medizinisches C-Bogen-Röntgengerät, umfassen.

Insbesondere vorteilhaft umfasst das Röntgenbildgebungsgerät, insbesondere CT-Gerät, einen photonenzählenden Röntgendetektor. Insbesondere kann ein direkt-konvertierender Röntgendetektor als photonenzählender Röntgendetektor ausgebildet sein, beispielsweise aufweisend CdTe, CdZnTe, CdTeSe, CdZnTeSe oder CdMnTe oder auch ein anderes Halbleitermaterial als Detektionsmaterial aufweisen.

Die Erfindung betifft außerdem ein Computerprogrammprodukt mit einem Computerprogramm, welches in einen Speicher einer zuvor beschriebenen Ansteuerungseinheit zur Ansteuerung eines Röntgenbildgebungsgeräts ladbar ist, mit Programmabschnitten, um alle Schritte des Verfahrens zur Ansteuerung eines Röntgenbildgebungsgeräts auszuführen, wenn die Programmabschnitte von der Ansteuerungseinheit ausgeführt werden.

Die Vorteile des vorgeschlagenen Computerprogrammprodukts bzw. computerlesbaren Speichermediumgs entsprechen im Wesentlichen den Vorteilen des vorgeschlagenen Verfahrens. Hierbei erwähnte Merkmale, Vorteile oder alternative Ausführungsformen können ebenso auch auf die anderen beanspruchten Gegenstände übertragen werden und umgekehrt.

Das Computerprogrammprodukt kann beispielsweise eine Software mit einem Quellcode, der noch kompiliert und gebunden oder nur interpretiert werden muss, oder einen ausführbaren Softwarecode umfassen, der zur Ausführung noch in die Ansteuerungseinheit zu laden ist. Durch das Computerprogrammprodukt kann das Verfahren Ansteuerung eines Röntgenbildgebungsgeräts mittels einer Ansteuerungseinheit schnell, identisch wiederholbar und robust ausgeführt werden. Computerprogrammprodukt ist so konfiguriert, dass es mittels der Ansteuerungseinheit die erfindungsgemäßen Verfahrensschritte ausführen kann.

Das Computerprogrammprodukt ist beispielsweise auf einem computerlesbaren Speichermedium gespeichert oder auf einem Netzwerk oder Server hinterlegt, von wo es in den Prozessor einer Ansteuerungseinheit geladen werden kann, der mit der Ansteuerungseinheit direkt verbunden oder als Teil der Ansteuerungseinheit ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in einer Ansteuerungseinheit ein erfindungsgemäßes Verfahren durchführen. Beispiele für elektronisch lesbare Datenträger sind eine DVD, ein Magnetband oder ein USB-Stick, auf welchem elektronisch lesbare Steuerinformationen, insbesondere Software, gespeichert ist. Wenn diese Steuerinformation von dem Datenträger gelesen und in eine Ansteuerungseinheit gespeichert werden, können alle erfindungsgemäßen Ausführungsformen der vorab beschriebenen Verfahren durchgeführt werden.

Eine weitgehend softwaremäßige Realisierung hat den Vorteil, dass schon bisher verwendete Ansteuerungseinheit auf einfache Weise durch ein Software-Update nachgerüstet werden können, um auf die erfindungsgemäße Weise zu arbeiten. Ein solches Computerprogrammprodukt kann neben dem Computerprogramm gegebenenfalls zusätzliche Bestandteile wie zum Beispiel eine Dokumentation und/oder zusätzliche Komponenten, sowie Hardware-Komponenten, wie zum Beispiel Hardware-Schlüssel (Dongles etc.) zur Nutzung der Software, umfassen.

Ausführungsbeispiele der Erfindung sind in den Zeichnungen dargestellt und werden im Folgenden näher beschrieben. In unterschiedlichen Figuren werden für gleiche Merkmale die gleichen Bezugszeichen verwendet. Es zeigen:
Fig. 1 ein schematisches Ablaufdiagramm des vorgeschlagenen Verfahrens zum Ansteuern eines Röntgenbildgebungsgeräts,
Fig. 2 eine schematische Darstellung eines Abbildungsbereichs an einem Objekt,
Fig. 3 eine schematische Darstellung einer Anordnung eines photonenzählenenden Röntgendetektors aufweisend eine Mehrzahl an Energieschwellen und eines Objekts,
Fig. 4 eine schematische Darstellung einer Ansteuerungseinheit, und
Fig. 5 eine schematische Darstellung eines Röntgenbildgebungsgeräts.

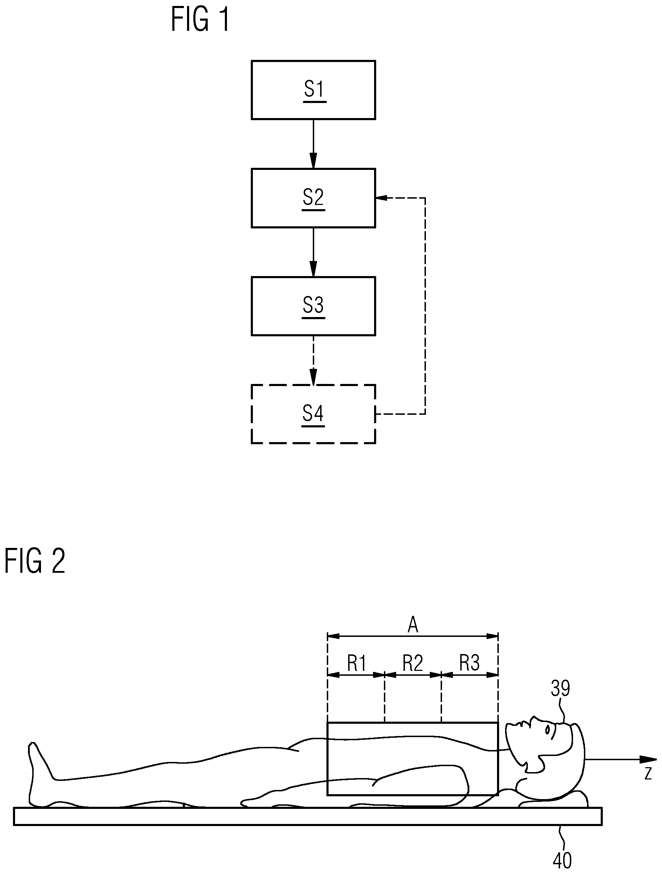

Fig. 1 zeigt ein schematisches Ablaufdiagramm des vorgeschlagenen Verfahrens zum Ansteuern eines Röntgenbildgebungsgeräts 32 aufweisend zumindest eine Röntgenquelle 37 und in Gegenüberstellung dazu einen photonenzählenden Röntgendetektor 36 zur Akquisition eines dreidimensionalen Objektbilddatensatzes eines Abbildungsbereichs A eines Objekts 39 umfassend die Schritte
- Bereitstellen S1 eines ersten spektralen Bilddatensatzes abbildend zumindest einen Teil des Abbildungsbereichs A des Objekts 39,
- Ableiten S2 einer Einstellung zumindest eines Ansteuerungsparameters T1, T2, T3 des Röntgenbildgebungsgeräts 32 basierend auf dem ersten spektralen Bilddatensatz für die Akquisition des dreidimensionalen Objektbilddatensatzes,
- Ansteuern S3 des Röntgenbildgebungsgeräts 32 zur Akquisition des dreidimensionalen Objektbilddatensatzes basierend auf der abgeleiteten Einstellung.

Weiterhin kann in Ausführungsvarianten des Verfahrens auch nach dem Ansteuern S3 wiederholt, beispielsweise ein zweiter oder dritter spektraler Bilddatensatz abbildend beispielsweise einen zweiten Teil des Abbildungsbereichs A des Objekts 39 oder auch den gleichen Teil des Abbildungsbereichs A zu einem anderen Zeitpunkt, bereitgestellt werden, S4, welcher anschließend für ein erneutes Ableiten S2 zur Verfügung gestellt wird.

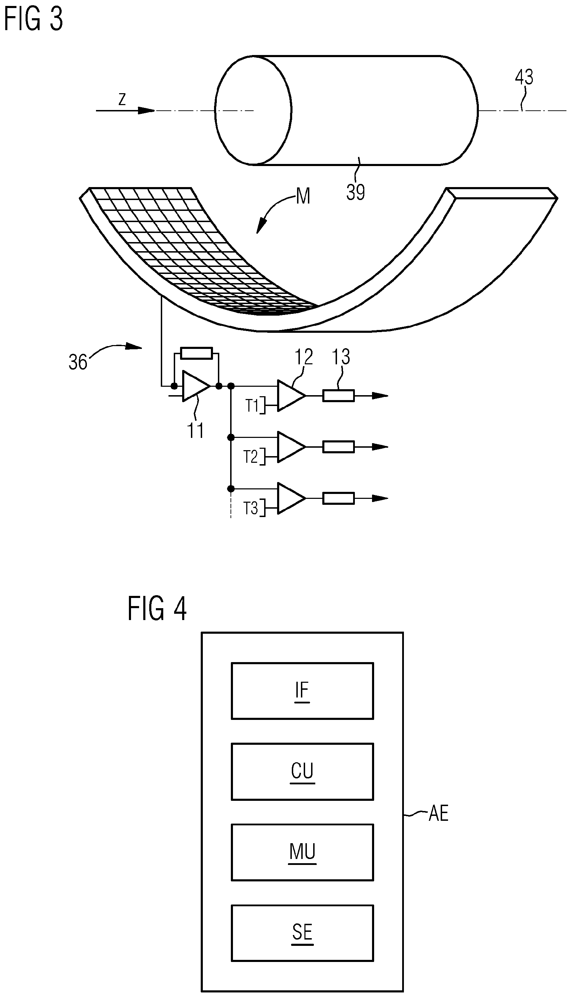

Das Röntgenbildgebungsgerät 32 ist beispielsweise als CT-Gerät oder C-Bogen-Röntgengerät ausgebildet. Insbesondere ist es ausgebildet einen 3D-Bilddatensatz, abbildend den Abbildungsbereich A des Objekts 39, zu akquirieren. Dies kann umfassen eine Mehrzahl an Projektionsdatensätzen zu erfassen, mittels welchem der Abbildungsbereich abgetastet wird. Beispielhaft zeigt Fig. 2 dabei einen Patienten 39 auf einer Patientenlagerungsvorrichtung 40 eines Röntgenbildgebungsgeräts, welcher entlang einer Längsachse z des Röntgenbildgebungsgeräts 32 ausgerichtet ist. Der mittels des Objektbilddatensatz abzubildende Abbildungsbereich A umfasst einen Teilbereich des Patienten 39, hier insbesondere den Thorax. Es können auch andere Abbildungsbereiche A bestimmt sein. Insbesondere umfasst das Röntgenbildgebungsgerät einen photonenzählenden Röntgendetektor, welcher ausgebildet ist spektral aufgelöste Messdaten basierend auf zumindest zwei Energieschwellwerten T1, T2, T3 (siehe auch Fig. 3) zu erfassen.

Der erste spektrale Bilddatensatz kann spektrale Information zumindest für den von ihm umfassten Teil des Abbildungsbereichs A in dem Sinne aufweisen, dass der erste spektrale Bilddatensatz auf Messdaten basiert, welche die Eigenschaften des Objekts 39 in Hinblick auf die Absorption von Röntgenstrahlung durch das Objekt 39 spektral aufgelöst, d.h. in Abhängigkeit zumindest zweier mittlerer Energien oder zumindest zweier Energiebereiche der Röntgenstrahlung, repräsentieren. Beispielsweise ist der erste spektrale Bilddatensatz mittels eines photonenzählenden Röntgendetektors 36 und in Abhängigkeit zumindest zweier Energieschwellwerte T1, T2, T3, in einer vorteilhaften Ausgestaltung zumindest dreier Energieschwellwerte, erfasst worden. Basierend auf dem spektral aufgelösten ersten Bilddatensatz kann dann verbessert auf eine erwartete spektrale Absorption von Röntgenstrahlung, insbesondere eine Schwächung und/oder auch eine Strahlaufhärtung durch das abbzubildende Objekt 39, und/oder eine zu erwartenden mittels des Röntgendetektors 36 gemessenen Statistik bei der Durchleuchtung des Objekts 39 mit Röntgenstrahlung bei der Akquisition des Objektbilddatensatzes mittels des photonenzählenden Röntgendetektors 36, insbesondere auch ortsaufgelöst, geschlossen werden.

Gemäß einem Aspekt der Erfindung kann der erste spektrale Bilddatensatz ein spektrales Topogramm, einen zuvor aufgenommenen, spektralen, dreidimensionalen Vorbilddatensatz und/oder einen während der Akquisition des dreidimensionalen Objektbilddatensatzes erfassten spektralen Projektionsdatensatz umfassen.

Basierend auf dem ersten spektralen Bilddatensatz wird eine Einstellung des zumindest einen Ansteuerungsparameter abgeleitet S2, welcher für die Ansteuerung des Röntgenbildgebungsgeräts 32 zur Akquisition des 3D-Objektbilddatensatzes eingesetzt wird. Die Einstellung kann für den gesamten Abbildungsbereich A, lediglich für den Teil des Abbildungsbereichs A, welcher vom erstem spektralen Bilddatensatz umfasst ist oder für einen zweiten Teil des Abbildungsbereichs A abgleitet werden, welcher ortsnah zum vom ersten spektralen Bilddatensatz umfassten ersten Teil liegt.

Die abgeleitete Einstellung für den zumindest einen Ansteuerungsparameter kann einen festen Wert oder auch einen Werteverlauf für den Ansteuerungsparameter umfassen, welcher bei der Akquisition des Objektbilddatensatzes angewandt werden soll und entsprechend bei der Ansteuerung des Röntgenbildgebungsgeräts 32 berücksichtigt wird. Der Ansteuerungsparameter kann insbesondere ein Ansteuerungsparameter der Röntgenröhre 37 oder des Röntgendetektors 36 umfassen. Insbesondere kann der zumindest eine Ansteuerungsparameter einen solchen Parameter umfassen, welcher direkten Einfluss auf eine im Röntgendetektor 36 gemessene Statistik und/oder die spektrale Separierung und/oder die gemessene spektrale Information hat. Insbesondere können auch Einstellungen mehrere Ansteuerungsparameter abgeleitet werden.

In einer vorteilhaften Ausbildung umfasst der zumindest eine Ansteuerungsparameter zumindest einen Energieschwellwert T1, T2, T3 des photonenzählenden Röntgendetektors 36 für die Akquisition des Objektbilddatensatzes. Alternativ oder zusätzlich kann auch eine Einstellung einer Röhrenspannung, eines Röhrenstroms oder einer Vorfilterung der Röntgenquelle 37 und/oder einer Rotationszeit oder eines relativen Vorschubs ("pitch") zwischen Objekt 39 und Röntgendetektor 36 während der Akquisition des Objektbilddatensatzes abgeleitet werden. Darüber hinaus kann es auch weitere Parameter des Röntgenbildgebungsgeräts 32 geben. Insbesondere können auch Einstellungen mehrerer Ansteuerungsparameter abgeleitet werden.

Das Ableiten der Einstellung des zumindest einen Ansteuerungsparameters basierend auf dem ersten spektralen Bilddatensatz ermöglicht das Bestimmen, insbesondere Abschätzen, einer erwarteten spektralen Absorption bei der Akquisition des Objektbilddatensatzes. Insbesondere kann basierend darauf eine optimierte Einstellung des zumindest einen Ansteuerungsparameters für die Akquisition des Objektbilddatensatzes, beispielsweise vorteilhaft auch ortsaufgelöst über den Abbildungsbereich, abgeleitet werden. Insbesondere kann dabei eine erwartete gemessene Statistik im Röntgendetektor 36 für die Akquisition des Objektbilddatensatz, insbesondere auch ortsaufgelöst über den Abbildungsbereich, verbessert abgeschätzt werden und darauf basierend die Einstellung des zumindest einen Ansteuerungsparameters optimiert werden.

Beispielsweise wird basierend auf dem ersten spektralen Bilddatensatz zumindest ein Energieschwellwert T1, T2, T3, vorzugsweise mehrere Energieschwellwerte T1, T2, T3, des Röntgendetektors 36 für die Akquisition des Objektbilddatensatzes abgeleitet, wobei verbessert sichergestellt wird, dass in Abhängigkeit der Energieschwellwerte T1, T2, T3 eine vorteilhafte Statistik mittels des Röntgendetektors 36 über den Abbildungsbereich gemessen wird.

Beim Ableiten kann vorzugsweise neben der durch den ersten spektralen Bilddatensatz zur Verfügung gestellten Objektinformation außerdem die dem Objektbilddatensatz zugrunde liegende klinische Bildgebungsanwendung bzw. der zugrunde liegende Bildgebungsgrund miteinbezogen werden. Vorteilhafterweise kann die erwartete Zielsetzung und ggf. mit der Bildgebungsanwendung einhergehende Randbedingungen der durchgeführten Bildgebung in das Ableiten der Einstellung des zumindest einen Ansteuerungsparameters eingehen und daraufhin optimiert werden.

Basierend auf der abgeleiteten Einstellung wird das Röntgenbildgebungsgerät 32 anschließend für die Akquisition des Objektbilddatensatzes angesteuert. Beispielsweise kann ein Steuersignal für das Röntgenbildgebungsgerät 32 und/oder einzelne Komponenten davon bereitgestellt werden, welches das Röntgenbildgebungsgerät 32 und/oder seine Komponenten in Bezug auf den Ansteuerungsparameter gemäß der abgeleiteten Einstellung anpasst.

Die abgeleitete Einstellung kann einen festen Wert für den zumindest einen Ansteuerungsparameter umfassen. Der eine Wert kann für den gesamten Abbildungsbereich A festgelegt sein.

Beispielsweise kann basierend auf den Informationen des ersten spektralen Bilddatensatzes ein fester Satz von Energieschwellwerten T1, T2, T3 für den Röntgendetektor 36 für den gesamten Abbildungsbereich A festgelegt werden, welcher berücksichtigt, dass im gesamten Abbildungs-bereich A eine geeignete Statistik in Abhängigkeit der Energieschwellwerte T1, T2, T3 am Röntgendetektor 36 gewährleistet ist. Dies kann genauso auch auf einen anderen Ansteuerungsparameter übertragen werden.

Beispielsweise kann ein spektrales Topogramm bereitgestellt sein, worauf basierend ortsaufgelöst die erwartete spektrale Absorption, insbesondere eine Schwächung und Strahlaufhärtung, im Abbildungsbereich A bestimmt werden kann. Anschließend kann eine Scangrund getriebene Bestimmung der erwarteten Statistik in Abhängigkeit der Energieschwellwerte T1, T2, T3 und eine entsprechende Optimierung der Energieschwellwerte T1, T2, T3 erfolgen. Beispielsweise wird anhand von Modellen eine feste, optimale Schwellenposition für den gesamten Abbildungsbereich A vordefiniert.

Der zumindest eine Ansteuerungsparameter kann jedoch während der Akquisition des dreidimensionalen Objektbilddatensatzes auch unterschiedliche Werte aufweisen. Dies kann insbesondere umfassen, dass der zumindest eine Ansteuerungsparameter zumindest basierend auf dem durch den ersten spektralen Bilddatensatz bereitgestellte Information während der Akquisition des Objektbilddatensatzes moduliert wird. Die Einstellung des zumindest einen Ansteuerungsparameters kann dadurch vorteilhaft auf die örtlichen Begebenheiten im Abbildungsbereich A abgestimmt werden.

Beispielsweise kann der Abbildungsbereich A in zumindest zwei Regionen R1, R2, R3 aufgeteilt sein und der zumindest eine Ansteuerungsparameter für die zumindest zwei Regionen R1, R2, R3 unterschiedliche Werte annehmen. Insbesondere können auch mehr als zwei Regionen R1, R2, R3 unterschieden und der zumindest eine Ansteuerungsparameter auf mehr als zwei Werte eingestellt werden.

Fig. 2 zeigt beispielhaft eine Unterteilung des Abbildungsbereichs A in drei Regionen R1, R2, R3, wobei für die drei Regionen R1, R2, R3 unterschiedliche Werte für den zumindest einen Ansteuerungsparameter abgeleitet sein können.

Insbesondere können solche Regionen R1, R2, R3 unterschieden werden, in welchen sich die spektrale Absorption voneinander unterscheidet, so dass ein angepasster Wert für den Ansteuerungsparameter zu vorteilhaften Ergebnissen führt. Weiterhin kann für unterschiedliche Regionen R1, R2, R3 eine unterschiedliche Zielsetzung der Anwendung und damit ein anderer optimierter Wert für den Ansteuerungsparameter einhergehen. Beispielsweise kann für die, insbesondere auch spektrale, Bildgebung eines bestimmten Organs ein anderer Wert für den Ansteuerungsparameter, beispielsweise andere Energieschellwerte T1, T2, T3, vorteilhaft sein als für eine Region abseits davon.

Daneben ist auch denkbar, dass ein kontinuierlicher Werteverlauf in Abhängigkeit der mittels des ersten spektralen Bilddatensatzes bereitgestellten Information für den zumindest einen Ansteuerungsparameter abgeleitet wird und derart eine kontinuierliche Anpassung des Ansteuerungsparameters während der Akquisition des Objektbilddatensatzes über den Abbildungsbereich A erreicht wird. Beispielsweise können Energieschwellwerte T1, T2, T3 kontinuierlich während der Akquisition des Objektbilddatensatzes angepasst werden.

Auch kann im Rahmen des Verfahrens vorgesehen sein, dass für die Akquisition des dreidimensionalen Objektbilddatensatzes eine Mehrzahl an Projektionsbilddatensätzen erfasst wird, wobei der Wert des zumindest einen Ansteuerungsparameter von Projektionsdatensatz zu Projektionsdatensatz angepasst wird. Das Anpassen kann dann umfassen, dass für jeden Projektionsdatensatz ein vorteilhafter Wert für den zumindest einen Ansteuerungsparameter abgeleitet wird, wodurch eine hochdynamische Modulation erreicht wird.

Auch kann vorgesehen sein, dass der zumindest eine Ansteuerungsparameter auch während einer Erfassung eines vom Objektbilddatensatz umfassten Projektionsdatensatzes unterschiedliche Werte aufweist.

Gemäß einem weiteren Aspekt der Erfindung umfasst der zumindest eine Ansteuerungsparameter einen Parameter des Röntgendetektors 36 und der photonenzählende Röntgendetektor 36 eine matrixartige Anordnung M einer Mehrzahl an Pixelelementen, wobei die Einstellung des zumindest einen Ansteuerungsparameters für Pixelelemente der Mehrzahl an Pixelelementen zumindest teilweise unterschiedlich ist.

Fig. 3 zeigt dazu beispielhaft eine rein schematische Darstellung einer Anordnung eines photonenzählenden Röntgendetektors 36 aufweisend eine Mehrzahl an Energieschwellen T1, T2, T3 und eines Objekts 39 in einem Röntgenbildgebungsgerät.

Die Anordnung ist in Anlehung einer Anordnung eines CT-Geräts veranschaulicht mit einem Objekt 39, welches entlang einer Rotationsachse 43 angeordnet ist, und um welches der Röntgendetektor 36 rotierbar angeordnet ist. Der Röntgendetektor 36 weist eine matrixartige Mehrzahl an Pixelelementen auf, welche eine ortsaufgelöste Detektion von Röntgenstrahlung nach Durchgang durch das Objekt 39 ermöglicht. Die dargestellte Anzahl an Pixelelementen ist dabei rein exemplarisch. Der Röntgendetektor kann insbesondere eine größere Anzahl an Pixelelementen aufweisen als hier dargestellt.

Der Röntgendetektor ist insbesondere als photonenzählendender, direkt-konvertierender Röntgendetektoren ausgebildet, wobei eintreffende Röntgenstrahlung bzw. Photonen durch ein geeignetes Konvertermaterial des Sensors in elektrische Pulse umgewandelt werden. Als Konvertermaterial können beispielsweise CdTe, CZT, HgI2, GaAs oder andere verwendet werden. Die elektrischen Pulse werden von einer Auswerteelektronik, beispielsweise einem integrierten Schaltkreis (Application Specific Integrated Circuit, ASIC), bewertet. Schematisch dargestellt ist dabei beispielhaft eine an ein Pixelelement des Senors anschließende Weiterverarbeitungskette der mittels des Sensor als Reaktion auf die detektierten Photonen erzeugten elektrischen Pulse in der Auswerteelektronik, welche in der Regel für jedes der Pixelelemente vorliegt, umfassend einen Signalverstärker 11, mehrere Komparatoren 12 und damit jeweils verbundene Zählelemente 13. Die erzeugten elektrischen Signale werden mittels des Signalverstärkers 11 verstärkt und ggf. geformt und anschließend mittels der Komparatoren mit mehreren Energieschwellwerten T1, T2, T3 verglichen. Übersteigt ein Signal einen Energieschwellwert T1, T2, T3 wird ein Zählereignis generiert, welches mittes des Zählelements 13 gezählt wird. Derart lässt sich ein einfallendes Röntgenspektrum spektral aufgelöst in Abhängigkeit der eingestellten Energieschwellwerte T1, T2, T3 vermessen, worauf basierend ein Bilddatensatz erfasst werden kann. Beispielhaft sind hier drei Energieschwellwerte angedeutet, es können jedoch auch mehr oder weniger sein.

Besonders vorteilhaft kann die Einstellung der jeweiligen Energieschwellwerte T1, T2, T3 des Röntgendetektors 36 mittels des vorgeschlagenen Verfahrens zumindest basierend auf dem ersten spektralen Bilddatensatz abgeleitet und für die Akquisition des Objektbilddatensatz angepasst werden.

Wie bereits zuvor beschrieben kann dabei die Einstellung des zumindest einen Ansteuerungsparameters für Pixelelemente der Mehrzahl an Pixelelementen zumindest teilweise unterschiedlich sein. Inbesondere kann dies umfassen, dass der Ansteuerungparameter für die Pixelelemente der Mehrzahl an Pixelelementen des photonenzählenden Röntgendetektors 36 räumlich getrennt voneinander eingestellbar und ggf. entsprechend räumlich getrennt auch während der Akquisition des Objektbilddatensatzes anpassbar ist. Beispielsweise werden im Schritt des Ableitens für verschiedene Pixelelemente der Mehrzahl verschiedene Einstellungen für die Anzahl an Energieschwellwerten für die Akquisition des Objektbilddatensatzes abgeleitet.

Dies kann umfassen, dass eine Ableitung jeweils pixelindividuell, d.h. für jedes Pixelelement separat, vorliegt und der Ansteuerungsparameter ggf. auch im Rahmen des vorgeschlagenen Verfahrens während der Akquisition des Objektbilddatensatzes pixelindividuell unterschiedliche Werte aufweist und/oder pixelindviduell angepasst wird. Dies kann gemäß einer vorteilhaften Variante auch umfassen, dass Pixelelemente in Gruppen, insbesondere vorzugsweise zeilenweise oder zeilengruppenweise, zusammengefasst werden, wobei eine Ableitung jeweils für die Gruppe an Pixelelementen zusammen erfolgt und der Ansteuerungsparameter ggf. auch im Rahmen des vorgeschlagenen Verfahrens während der Akquisition des Objektbilddatensatzes gruppenweise unterschiedliche Werte aufweist und/oder entsprechend gruppenweise angepasst wird.

Das heißt, für den zumindest einen Ansteuerungsparameter kann eine innerhalb der Pixelmatrix M variierende Einstellung, inbesondere beispielsweise zeilenweise oder zeilengruppeweise entlang einer Längsachse z des Röntgenbildgebungsgerätes 32, und darüberhinaus auch eine jeweilige zeitliche Modulation der Werte während der Akquisition des Objektbilddatensatzes erfolgen.

Beispielsweise kann ein kontinuierliches zeilen- oder zeilengruppenweises Verschieben des oder der Energieschwellwerte T1, T2, T3 während der Akquisition des Objektbilddatensatzes, insbesondere in Abhängigkeit eines relativen Veschubs zwischen Röntgendetektor 36 und Objekt 39, vorgesehen sein.

Beispielsweise kann der Abbildungsbereich A in mehrere Regionen R1, R2, R3 aufgeteilt sein, wobei in Abhängigkeit der relativen Position eines jeweiligen Pixelelements aus der Mehrzahl an Pixelelementen zu den Regionen der oder die Energieschwellwerte T1, T2, T3 des jeweilligen Pixelelements angepasst werden. Beispielsweise bildet ein erster Teil der Pixelmatrix eine erste Region an, wohingegen ein zweiter Teil der Pixelmatrix eine zweite Region abbildet und wobei der oder die Energieschwellwerte T1, T2, T3 des ersten Teils der Pixelmatrix einen anderen an die entsprechende Region R1, R2, R3 angepassten Wert aufweisen als der oder die Energieschwellwerte T1, T2, T3 des zweiten Teils. Die Zugehörigkeit zu einem ersten oder zweiten Teil der Pixelmatrix kann sich dabei außerdem zeitlich mit einem Verschub zwischen Objekt 39 und Röntgendetektor 36 verändern.

Besonders vorteilhaft ist eine zuvor beschriebene räumliche Anpassbarkeit innerhalb der Pixelmatrix in Kombination mit einer großflächige Ausdehnung des Röntgendetektors 36 entlang der z-Richtung, inbesondere mit einer Ausdehnung von mindestens 6 cm. Insbesondere beim Einsatz solch großflächiger Röntgendetektoren 36 ist eine räumlich getrennte Einstellung innerhalb der Pixelmatrix M besonders vorteilhaft für die Akquisition des Objektbilddatensatzes einsetzbar. Solche Röntgendetektoren überstrecken bei der Akquisition jeweils einen Bereich des Objekts 39, in welchem in unterschiedlichen Teilbereichen bereits deutlich unterschiedliche Objekteigenschaften vorliegen können. Eine räumlich getrennte Ableitung und Einstellbarkeit ermöglicht vorteilhaft dem Rechnung zu tragen und eine gezielte und optimierte Einstellung des zumindest einen Ansteuerungsparameter über den Röntgendetektor 36 zu gewährleisten.

Auch stellt dabei die Kombination mit dem Betrieb des Röntgenbildgebungsgerät 32 in einem sequentiellen Scanmodus eine weiterhin vorteilhafte Verfahrensvariante dar, wobei der zumindest eine Ansteuerungsparameter an einer ersten relativen Position zwischen Objekt 39 und Röntgendetektor 36 entlang einer Längsachse des Röntgenbildgebungsgeräts 32 und an einer zweiten relativen Position zwischen Objekt 32 und Röntgendetektor 36 entlang der Längsachse z unterschiedliche Werte aufweist. Bei einer Kombination mit einer räumlich aufgelösten Einstellbarkeit des zumindest einen Ansteuerungsparameters, insbesondere der Energieschwellwerte T1, T2, T3 innerhalb der Pixelmatrix M des Röntgendetektors 36 lässt sich durch einen sequentiellen Scanmodus beispielsweise eine vereinfachte Zuordnung und Bildrekonstruktion trotz der erhöhten Komplexität der Messdaten im Gegensatz, beispielsweise, zu einer Kombination mit einer Spiralabtastung erreichen, da hier nicht zusätzlich außerdem ein kontinuierlicher Verschub des Objekts 39 berücksichtigt werden muss.

Im Rahmen des vorgeschlagenen Verfahrens ist eine Kombination der Ansteuerung verschiedener Ansteuerungsparameter denkbar. Beispielsweise kann je nach System und Aufgabe eine hierauf basierende Optimierung verschiedener Parameter vorgesehen sein, beispielsweise auch eine flexible/dynamische Modulation während des Scans von Projektion zu Projektion, basierend auf der vorangegangenen Projektion. Beispielsweise könnte eine Röhrenspannungsanpassung über der Schulter, ein zusätzliches Spektrum über einer interessierenden Struktur bei einem Dual Source CT ggf. mit unterschiedlichen Röhrenspannungen, eine Anpassung von Pitch und Rotationszeit für bessere Projektionen im Sinne einer besseren Statistik für die jeweilige Task vorgesehen sein und in Kombination beispielweise mit einer Optimierung der Energieschwellwerte vorliegen.

Fig. 4 zeigt eine schematische Darstellung einer Ansteuerungseinheit AE, welche zur Ausführung eines vorgeschlagenen Verfahrens und seiner Aspekte ausgebildet ist. Die Bereitstellungseinheit AE umfasst eine Schnittstelle IF, eine Recheneinheit CU und eine Speichereinheit MU. Außerdem umfasst die Ansteuerungseinheit AE eine Steuereinheit SE.

Die Komponenten der Ansteuerungseinheit AE können miteinander verbunden sein, um einen effizienten Datenaustausch zu ermöglichen. Die Schnittstelle IF kann direkt mit der Recheneinheit CU verbunden sein, welche wiederum mit der Speichereinheit MU verbunden sein kann. Diese Anordnung kann einen Informationsfluss von der Schnittstelle IF über die Recheneinheit CU zur Speichereinheit MU ermöglichen. Gleiches gilt für die Steuereinheit SE.

Die Ansteuerungseinheit AE kann für die Datenverarbeitung und -speicherung ausgelegt sein. Die Schnittstelle IF kann dabei als Schnittstelle für Ein-/Ausgabeoperationen dienen, die Recheneinheit CU kann Berechnungsaufgaben durchführen und die Speichereinheit MU kann Daten oder Ergebnisse speichern.

Die Schnittstelle kann insbesondere dazu ausgebildet sein, einen ersten spektralen Bilddatensatz bereitzustellen. Die Recheneinheit CU kann ausgebildet sein die Schritte des Ableitens des vorgeschlagenen Verfahrens und entsprechende Schritte seiner Ausführungsvarianten auszuführen.

Die Speichereinheit MU kann dazu ausgebildet sein, einen ersten spektralen Bilddatensatz zu speichern. Die Speichereinheit MU kann verschiedene Speichertechnologien verwenden, um eine effiziente Verwaltung und schnellen Zugriff auf die gespeicherten Daten zu ermöglichen.

Die Steuereinheit SE kann dazu ausgebildet sein, basierend auf der abgeleiteten Einstellung ein Steuersignal für Ansteuerung des Röntgenbildgebungsgeräts 32 auszugeben.

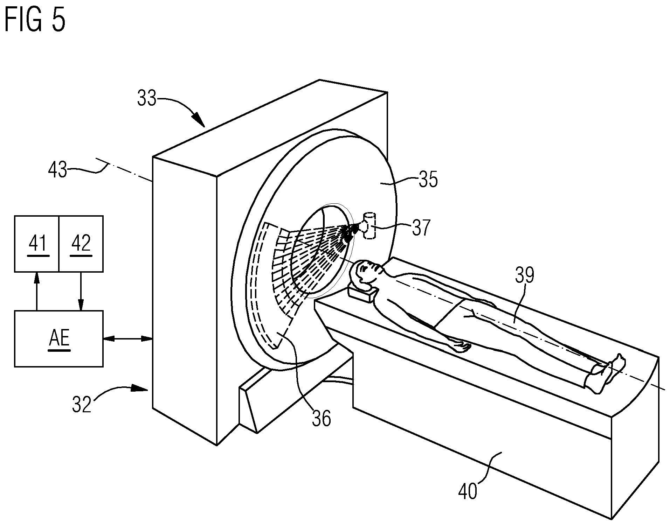

Fig. 5 zeigt eine schematische Darstellung eines Röntgenbildgebungsgeräts 32.

Das medizinische Röntgenbildgebungsgerät 32 ist in diesem Beispiel insbesondere ein CT-Gerät umfassend eine die Strahlenquelle, hier insbesondere Röntgenquelle 37, einen Strahlendetektor, hier insbesondere Röntgendetektor 36, und eine Ansteuerungseinheit AE. Dabei können die Röntgenquelle 37 und der Röntgendetektor 36 in Gegenüberstellung zueinander angeordnet sein. Die Röntgenquelle 37 kann dazu ausgebildet sein, den Röntgendetektor 36 entlang einer Röntgenstrahleneinfallsrichtung mit Röntgenstrahlung zu beleuchten. Der Röntgendetektor 36 ist insebsondere ein photonenzählender, direkt-konvertierender Röntgendetektor, beispielsweise aufweisend CdTe, CdZnTe, CdTeSe, CdZnTeSe oder CdMnTe oder auch ein anderes Halbleitermaterial als Detektionsmaterial.

Das CT-Gerät 32 kann eine Gantry 33 mit einem Rotor 35 umfassen. Die Röntgenquelle 37 und der Röntgendetektor 36 können in definierter Anordnung an dem Rotor 35 angeordnet sein, insbesondere in den Rotor 35 integriert oder an dem Rotor 35 befestigt sein. Der Rotor 35 kann um eine Rotationsachse 43 drehbar gelagert sein. Die Längsachse z des Röntgenbildgebungsgeräts 32 verläuft parallel zur Drehachse 43. Das CT-Gerät könnte auch als Dual Source-Gerät aufweisend zwei versetzt zueinander angeordnete Röntgenquellen-Röntgendetektor-Kombinationen ausgebildet sein.

Das abzubildendes Untersuchungsobjekt 39, hier ein menschlicher Patient, ist auf der Patientenlagerungsvorrichtung 40 gelagert und entlang der Rotationsachse 43 durch die Gantry 32 hindurch bewegbar. Zur Steuerung des CT-Geräts 32 und zur Berechnung von Schnittbildern bzw. Volumenbildern des Untersuchungsobjekts 39 kann die Ansteuerungseinheit AE verwendet werden. Die Ansteuereinheit AE ist insbesondere zur Ausführung eines zuvor vorgeschlagenen Verfahrens zur Ansteuerung eines Röntgenbildgebungsgeräts 32 und seiner Aspekte ausgebildet.

Die Ansteuerungseinheit AE ist mit einer Eingabeeinheit 42, beispielsweise eine Tastatur und/oder einer Maus, und eine Darstellungseinheit 41, beispielsweise einen Monitor und/oder Display, verbunden. Die Eingabeeinheit 42 kann in die Darstellungseinheit 41 integriert sein, beispielsweise bei einem kapazitiven und/oder resistiven Eingabedisplay. Dabei kann durch eine Eingabe eines medizinischen Bedienpersonals an der Eingabeeinheit 42 eine Steuerung der Ansteuerungseinheit AE ermöglicht werden. Hierfür kann die Eingabeeinheit 42 beispielsweise ein Signal an die Ansteuerungseinheit AE senden. Die Darstellungseinheit 41 kann vorteilhafterweise zur Anzeige von 2D- und 3D-Bilddatensätzen und daraus abgeleiteter Informationen und Darstellungen, beispielsweise Querschnittsdarstellungen, ausgebildet sein. Hierfür kann Ansteuerungseinheit AE ein Signal an die Darstellungseinheit 41 senden. Weiterhin kann mittels der Eingabeeinheit 42 und ggf. der Darstellungseinheit 41 eine manuelle Eingabe eines medizinischen Bedienpersonals ermöglicht werden.

Die in den beschriebenen Figuren enthaltenen schematischen Darstellungen bilden keinerlei Maßstab oder Größenverhältnisse ab.

Es wird abschließend noch einmal darauf hingewiesen, dass es sich bei den vorhergehend detailliert beschriebenen Verfahren sowie bei den dargestellten Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Element" nicht aus, dass die betreffenden Komponenten aus mehreren zusammenwirkenden Teil-Komponenten bestehen, die gegebenenfalls auch räumlich verteilt sein können.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

## Patentansprüche

1. Verfahren zur Ansteuerung eines Röntgenbildgebungsgeräts (32) aufweisend zumindest eine Röntgenquelle (37) und in Gegenüberstellung dazu einen photonenzählenden Röntgendetektor (36) zur Akquisition eines dreidimensionalen Objektbilddatensatzes eines Abbildungsbereichs (A) eines Objekts (39) umfassend die Schritte
- Bereitstellen (S1) eines ersten spektralen Bilddatensatzes abbildend zumindest einen Teil des Abbildungsbereichs (A) des Objekts (39),
- Ableiten (S2) einer Einstellung zumindest eines Ansteuerungsparameters (T1, T2, T3) des Röntgenbildgebungsgeräts (32) basierend auf dem ersten spektralen Bilddatensatz für die Akquisition des dreidimensionalen Objektbilddatensatzes,
- Ansteuern (S3) des Röntgenbildgebungsgeräts (32) zur Akquisition des dreidimensionalen Objektbilddatensatzes basierend auf der abgeleiteten Einstellung.

2. Verfahren nach Anspruch 1, wobei der erste spektrale Bilddatensatz ein spektrales Topogramm, einen zuvor aufgenommenen, spektralen, dreidimensionalen Vorbilddatensatz und/oder einen während der Akquisition des dreidimensionalen Objektbilddatensatzes erfassten spektralen Projektionsdatensatz umfasst.

3. Verfahren nach einem der vorangehenden Ansprüche, wobei der erste spektrale Bilddatensatz auf energieaufgelösten Messdaten basierend auf mindestens drei Energieschwellwerten (T1, T2, T3) basiert.

4. Verfahren nach einem der vorangehenden Ansprüche, wobei das Ableiten eine Bestimmung der erwarteten spektralen Absorption im vom ersten spektralen Bilddatensatz umfassten Teil des Abbildungsbereichs (A) umfasst.

5. Verfahren nach einem der vorangehenden Ansprüche, wobei der zumindest eine Ansteuerungsparameter einen Energieschwellwert (T1, T2, T3) des photonenzählenden Röntgendetektors (36) für die Akquisition des Objektbilddatensatzes und/oder eine Röhrenspannung, einen Röhrenstrom oder eine Vorfilterung der Röntgenquelle (37) und/oder eine Rotationszeit oder einen relativen Vorschub zwischen Objekt (39) und Röntgendetektor (36) während der Akquisition des Objektbilddatensatzes umfasst.

6. Verfahren nach einem der vorangehenden Ansprüche, wobei der zumindest eine Ansteuerungsparameter während der Akquisition des dreidimensionalen Objektbilddatensatzes unterschiedliche Werte aufweist.

7. Verfahren nach Anspruch 6, wobei der Abbildungsbereich (A) in zumindest zwei Regionen (R1,R2,R3) aufgeteilt ist und der zumindest eine Ansteuerungsparameter für die zumindest zwei Regionen (R1,R2,R3) unterschiedliche Werte aufweist.

8. Verfahren nach Anspruch 6, wobei für die Akquisition des dreidimensionalen Objektbilddatensatzes eine Mehrzahl an Projektionsdatensätzen erfasst wird und wobei der Wert des zumindest einen Ansteuerungsparameters von Projektionsdatensatz zu Projektionsdatensatz angepasst wird.

9. Verfahren nach einem der vorangehenden Ansprüche, wobei der zumindest eine Ansteuerungsparameter während einer Erfassung eines vom Objektbilddatensatz umfassten Projektionsdatensatzes unterschiedliche Werte aufweist.

10. Verfahren nach einem der vorangehenden Ansprüche, wobei das Röntgenbildgebungsgerät (32) für die Akquisition des Objektbilddatensatzes in einem sequentiellen Scanmodus betrieben wird und der zumindest eine Ansteuerungsparameter an einer ersten relativen Position zwischen Objekt (39) und Röntgendetektor (36) entlang einer Längsachse (z) des Röntgenbildgebungsgeräts (32) und an einer zweiten relativen Position zwischen Objekt (39) und Röntgendetektor (36) entlang der Längsachse (z) unterschiedliche Werte aufweist.

11. Verfahren nach einem der vorangehenden Ansprüche, wobei der photonenzählende Röntgendetektor (36) eine matrixartige Anordnung (M) einer Mehrzahl an Pixelelementen umfasst und wobei während der Akquisition des Objektbilddatensatzes die Einstellung des zumindest einen Ansteuerungsparameters für Pixelelemente der Mehrzahl an Pixelelementen zumindest teilweise unterschiedlich ist.

12. Verfahren nach Anspruch 11, wobei die Einstellung des zumindest einen Ansteuerungsparameters entlang einer Längsachse (z) des Röntgenbildgebungsgeräts (32) innerhalb der matrixartigen Anordnung der Mehrzahl an Pixelelementen variiert.

13. Verfahren nach einem der vorangehenden Ansprüche, wobei der photonenzählende Röntgendetektor (36) entlang einer Längsachse (z) des Röntgenbildgebungsgeräts (36) eine Ausdehnung von mindestens 6 cm aufweist.

14. Ansteuerungseinheit (AE) zur Ansteuerung eines Röntgenbildgebungsgeräts (32) aufweisend zumindest eine Röntgenquelle (37) und in Gegenüberstellung dazu einen photonenzählenden Röntgendetektor (36) zur Akquisition eines dreidimensionalen Objektbilddatensatzes eines Abbildungsbereichs (A) eines Objekts (39), welche zur Ausführung eines Verfahrens nach einem der vorangehenden Ansprüche ausgebildet ist

15. Röntgenbildgebungsgerät (32) umfassend eine Ansteuerungseinheit (AE) nach Anspruch 14.

16. Computerprogrammprodukt mit einem Computerprogramm, welches in einen Speicher (MU) einer Ansteuerungseinheit (AE) zur Ansteuerung eines Röntgenbildgebungsgeräts (32) nach Anspruch 14 ladbar ist, mit Programmabschnitten, um alle Schritte des Verfahrens nach einem der Ansprüche 1 bis 13 auszuführen, wenn die Programmabschnitte von der Ansteuerungseinheit (AE) ausgeführt werden.
